# NEW EUROPEAN PATENT SPECIFICATION

(11) **EP 2 089 111 B2**
(45) Date of publication and mention of the opposition decision: **04.09.2013**
(45) Mention of the grant of the patent: 24.02.2010
(21) Application number: 07847667.8
(22) Date of filing: 03.12.2007
(51) Int. Cl.: A61Q 5/02, A61Q 19/10, A61K 8/02, A61K 8/46, C11D 1/37, C11D 1/83, C11D 1/94, C11D 17/00

(54) **PROCESS FOR MANUFACTURING CONCENTRATED SURFACTANT COMPOSITIONS**
KONZENTRIERTE TENSIDZUSAMMENSETZUNGEN
COMPOSITIONS TENSIOACTIVES CONCENTRÉES

(30) Priority: 08.12.2006 EP 06125743
(43) Date of publication of application: 19.08.2009
(73) Proprietor: Unilever PLC, London EC4Y 0DY (GB); Unilever N.V., 3013 AL Rotterdam (NL)
(72) Inventor: BASAPPA, Geetha, Bedford Bedfordshire MK44 1LQ (GB); BELMAR, Maria Teresa, Bedford Bedfordshire MK44 1LQ (GB); TELFORD, Julia Helen, Bedford Bedfordshire MK44 1LQ (GB)
(74) Representative: Bristow, Stephen Robert
(86) International application number: PCT/EP2007/063156
(87) International publication number: WO 2008/068222

(56) References cited:
- EP-A- 0 922 754
- EP-A1- 1 696 023
- EP-A2- 1 225 214
- WO-A-99/09944
- WO-A1-94/18946
- WO-A2-01/25378
- DE-A1- 19 641 280
- DE-A1- 19 646 882
- DE-A1- 19 929 511
- DE-A1- 19 944 544
- DE-A1- 19 950 925
- FR-A1- 2 398 797
- FR-A1- 2 826 017
- GB-A- 2 013 235
- GB-A- 2 021 141
- GB-A- 2 022 125
- US-A- 4 898 690
- US-A- 5 066 425
- US-A1- 2005 143 277
- MCCUTCHEON'S: 'Emulsifiers & detergents international edition', vol. 1, 1999, THE MANUFACTURING CONFECTIONER PUBLISHING CO., GLEN ROCK page 55
- HENKEL KGAA: 'Texapon N70' COSPHA no. 6, September 1996,
- FARN, RICHARD, J.: 'Chemistry and technology of surfactants', 2006, BLACKWELL PUBLISHING, OXFORD pages 1 - 338
- PROCTER & GAMBLE: 'Process for manufacturing concentrated surfactant compositions' TECHNICAL REPORT 18 July 2012, pages 1 - 2

## Description

The present invention relates to a method of manufacture of a composition comprising a high amount of a specific surfactant mixture and water.

Concentrated formulations comprising surfactants as well as mixtures of surfactants are usually provided to reduce the transport costs as well as to reduce storage capacities. Usually the commonly known concentrates comprise all essential ingredients for producing, for example, a personal care composition with the exception of solvent (which is usually water).

The goal of the present invention is to provide a highly concentrated surfactant composition which is stable, which can serve as a base composition (skeleton composition) and which can be handled reasonably easily and comprises two surfactants and water only.

FR 2 826 017 A1 discloses a mixture of surfactants comprising an alkyl and/or alkenyl oligoglycoside, betaine and an alkyl ether sulphate

EP 1 225 214 A2 discloses a detergent composition which foams well and is not slimy containing an alkali metal taurate salt or an organic alkali taurate salt of N-acylmethyltaurate, N-acyltaurine, alkylsulphuric ester, alkyl ether sulphuric ester or alkylsulphonic acid.

DE 199 44 544 A1 discloses a surfactant mixture comprising an alkyloligoglycoside and an alkyl- and/or alkenylether sulphate.

FR 2 398 797 A1 discloses concentrated aqueous surfactant compositions comprising mixtures of different surfactants for use in aqueous systems.

WO 99/09944 A1 discloses aqueous nacreous lustre dispersions containing fatty acid polyglycol ester sulphate, emulsifiers, nacreous lustre waxes and polyols.

DE 196 41 280 A1 discloses waterborne pearlescent dispersions comprising monoglyceride (ether)sulphate, emulsifiers and polyols.

EP 0 922 754 A1 discloses pearlescent compositions comprising a fatty acid, an alkylether sulphate or acylisethionate, and water.

DE 196 46 882 A1 discloses an aqueous polyol free pearlescing concentrate based on selected surface-active emulsifiers and pearlescent waxes.

### Description of the Invention

The present invention relates to a process for manufacturing a composition, the composition comprising:
(i) 50-95% by weight, based on the total weight of the composition, of
   (ia) an alkyl ether sulphate and/or salt thereof, and
   (ib) at least one further surfactant which is an anionic and/or amphoteric surfactant, and
   (ic) optionally one or more non-ionic surfactants; and
(ii) 5 - 50 % by weight, based on the total weight of the composition, of water,
wherein the alkyl ether sulphate and/or salt thereof of (ia) and the anionic surfactant of (ib) are not identical surfactants, the process comprising the steps of:
(a) providing the alkyl ether sulphate or salt thereof in the form of a mixture with no more than 35% by weight, based on the total weight of the mixture, of water; then
(b) adding to and mixing with the mixture the at least one further surfactant chosen from the group consisting of anionic and/or amphoteric surfactants in substantially anhydrous form and the optional one or more non-ionic surfactants in substantially anhydrous form thereby to form the composition,
wherein the alkyl ether sulphate or salt thereof remains in a liquid crystalline lamellar phase or lamellar gel phase throughout the process.

Preferred compositions comprise 50 - 90 %, preferably 60 - 85 % by weight of (i). As a consequence thereof preferred compositions comprise 10 - 50 %, preferably 15 - 40 % by weight, based on the total weight of the composition, of water.

A further embodiment of the present invention is a composition where the ratio of (ia) : [(ib) + (ic)] is 1:2 to 40:1, preferably 1: 1 to 30:1.

Preferred compositions comprise ≤ 10 % by weight, based on the total amount of the composition, of non-ionic surfactants.

The compositions have the advantage that without using any further additives, the compositions show good stability as well as good handling properties. Without the need to include any further additives (such as, for example, preservatives), it opens a wide field of interesting applications and uses. A user can, for example, choose to add a preservative (or other ingredients) or not. The user can be found in industry as well as in private households.

The composition is preferably used as a base composition for a personal care composition. Preferred personal care compositions are body washes (such as for shower or bath) and/or shampoos.

The compositions preferably have a pH ≥ 5, preferably ≥ 5.5.

The composition preferably excludes added preservative, thus the composition may contain traces of preservatives which can be found in the commercially available surfactants and which cannot be removed completely. The amount of preservative in the composition is usually less than 0.1 % by weight of the composition.

The term preservative is to be understood as a natural or synthetic chemical that is added to products to retard spoilage, whether from microbial growth, or undesirable chemical changes. A distinction is sometimes made between anti-microbial preservatives which function by inhibiting the growth of bacteria and fungi, and antioxidants such as oxygen absorbers, which inhibit the oxidation of food constituents. Common anti-microbial preservatives include sodium nitrate, sodium nitrite, sulphites (sulfur dioxide, sodium bisulfite, potassium hydrogen sulfite) and disodium EDTA. Antioxidants include BHA and BHT. Other preservatives include formaldehyde (usually in solution), glutaraldehyde, diatomaceous earth (kills insects), ethanol, dimethyl dicarbonate and methylchloroisothiazolinone. Further preservatives include 2-bromo-2-nitropropane-1,3-diol, phenoxy ethanol citric acid, tartaric acid, phosphoric acid, iminodiacetic acid, nitrilotriacetic acid, hydroxyethyleneaminodiacetic acid and ethylenediaminetetraacetic acid and salts thereof; para-hydroxybenzoates such as butyl paraben, methyl paraben and propyl paraben; imidazolines (e.g. imidiazolinylurea), triclosan, hydantoins (e.g. dimethyloldimethylhydantoin), isothiazolidinone compounds; and mixtures thereof. Commercially available preservatives include Kathon^{®} CG, Kathon^{®} CGII and Myacide^{®}.

A further preferred embodiment relates to a composition excluding a pearlescent agent or an opacifying agent. Examples of a pearlescent or an opacifying agent include, but are not limited to: mono- or diesters of (a) fatty acids having 16 to 22 carbon atoms and (b) either ethylene or propylene glycol; mono- or diesters of (a) fatty acids having 16 to 22 carbon atoms (b) a polyalkylene glycol of the formula HO-(JO)ₐ-H, wherein J is an alkylene group having 2 to 3 carbon atoms, and a is 2 or 3, and fatty alcohols containing 16 to 22 carbon atoms; fatty esters of the formula KCOOCH₂L, wherein K and L independently contain 15 to 21 carbon atoms; inorganic solids insoluble in the composition; and mixtures thereof.

The compositions always comprise alkyl ether sulphates or salts thereof. Preferred alkyl ether sulphates salts are those of formula

RO-(CH₂CH₂O)ₓSO₃M,

wherein R is an alkyl or alkenyl group of 8 to 24 carbon atoms,
x is an integer having a value of 1 to 10, and
M is a cation such as ammonium, alkanolamines such as triethanolamine, monovalent metals such as sodium and potassium, and polyvalent metal cations such as magnesium and calcium.

Preferably, R has 8 to 18 carbon atoms, more preferably 10 to 16 carbon atoms, even more preferably 12 to 14 carbon atoms. The alkyl ether sulphate salts are typically made as condensation products of ethylene oxide and monohydric alcohols having 8 to 24 carbon atoms. The alcohols can be synthetic or they can be derived from fats, e.g. coconut oil, palm kernel oil and tallow. Lauryl alcohol and other straight chain alcohols derived from coconut oil or palm kernel oil are preferred. Such alcohols are reacted with between 0 and 10, preferably 1 to 5, more preferably 2 to 3, molar proportions of ethylene oxide, and the resulting mixture of molecular species having, for example, an average of 3 moles of ethylene oxide per mole of alcohol, is sulphated and neutralized.

Specific non-limiting examples of alkyl ether sulphate salts which may be used in the compositions include sodium and ammonium salts of coconut alkyl triethylene glycol ether sulphate, tallow alkyl diethylene glycol ether sulphate, tallow alkyl triethylene glycol ether sulphate and tallow alkyl hexaethylene sulphate. The preferred alkyl ether sulphate salt is sodium laureth sulphate.

As component (ib) at least one anionic and/or at least one amphoteric surfactant is present. The anionic surfactant may be selected from the group consisting of alkyl ether sulphate, alkyl sulphates, ethoxylated alkyl sulphates, alkyl ethoxycarboxylates, methyl acyl taurates, fatty acyl glycinates, N-acyl glutamates, acyl isethionates, alkyl sulfosuccinates, alkyl ethoxysulphosuccinates, alpha sulphonated fatty acids, esters of alpha sulphonated fatty acids, alkyl phosphate esters, ethoxylated alkyl phosphate esters, acyl sarcosinates, acyl aspartates, alkoxy cocamide carboxylates, (ethoxylated) alkanolamide sulphosuccinates, ethoxylated alkyl citrate sulphosuccinates, acyl ethylene diamine triacetates, acylhydroxyethyl isethionates, acyl amide alkoxy sulphates, linear alkyl benzene sulphonates, paraffin sulphonates, alpha olefin sulphonates, fatty acid/protein condensates, salts thereof, soaps such as ammonium, magnesium, potassium, triethanolamine and sodium salts of lauric acid, myristic acid and palmitic acid, and mixtures thereof. Alkyl and/or acyl chain lengths for these surfactants are C₆ -C₂₂, preferably C₁₂ -C₁₈ more preferably C₁₂-C₁₄.

Amphoteric surfactants suitable for use in the composition are well known in the art, and include those surfactants broadly described as derivatives of an aliphatic quaternary ammonium, phosphonium, or sulphonium compound, in which the aliphatic radicals can be straight or branched chain, and wherein one aliphatic substituent contains 8 to 18 carbon atoms and the same or different aliphatic substituent contains an anionic group such as a carboxy, a sulphonate, a sulphate, a phosphate or a phosphonate group. Zwitterionics such as betaines are preferred. A particularly preferred betaine is cocamidopropyl betaine.

The non-ionic surfactant as component (ic) can be chosen from any commonly known non-ionic surfactant, such as alkyl poly(ethylene oxide) alkyl polyglucosides (such as octyl glucoside and decyl maltoside), fatty alcohols (such as cetyl alcohol and oleyl alcohol), cocamide MEA, cocamide DEA and cocamide TEA.

The composition may be used in a personal care composition. Preferably the personal care compositions are body washes (for shower and bath) and shampoos. The personal care composition is produced by dilution and addition of optional further ingredients. If further ingredients (additives) are necessary in the personal care composition it is also possible to add them before and/or during and/or after the dilution process. The composition can comprise one or more additive. Such additives can include cationic polymers, particles, conditioning agents (hydrocarbon oils, fatty esters, silicones), anti-dandruff agents, suspending agents, viscosity modifiers, dyes, non-volatile solvents or diluents (water soluble and insoluble), pearlescent aids, foam boosters, additional surfactants or nonionic cosurfactants, pediculocides, pH adjusting agents, perfumes, preservatives, chelants, proteins, skin active agents, sunscreens, UV absorbers and vitamins. When a composition comprises at least one of these ingredients, then the amount is in the range 1 to 20 % by weight, based on the total weight of the composition. The amount is dependent on the degree of dilution.

Preferably the composition is translucent.

For ease of handling (e.g. pouring out of a container or pumping through pipes) it is important that the composition as described above is formulated such that it forms a liquid crystalline lamellar and/or lamellar gel phase and thus not a hexagonal phase.

By substantially anhydrous is meant less than 10%, preferably less than 5 %, more preferably less than 1% and most preferably less than 0.1 % by weight, based on the weight of the surfactant, of water.

By manufacturing the compositions in this manner, the alkyl ether sulphate or salt thereof always remains in a liquid crystalline lamellar phase or lamellar gel phase throughout the process. The advantage of this is that the viscosity of the composition always remains at a level where the handling and processing of the composition is easier. If the percentage amount of water is allowed to rise, for example through addition of an aqueous solution of the anionic and/or amphoteric surfactants or the optional one or more non-ionic surfactants, the composition will enter the hexagonal phase which has been observed to have significantly higher viscosities such that handling and processing of the composition becomes much more difficult. The various phases may be identified using optical microscopy.

Preferably the mixture of alkyl ether sulphate or salt thereof and water comprises no more than 30%, preferably no more than 25% and more preferably no more than 20% by weight, based on the total weight of the mixture, of water.

A process for manufacturing a personal care composition is provided comprising the steps of:
(a) providing a composition from the process according to the invention; then
(b) diluting the composition with water using a high shear in-line mixer thereby to arrive at the personal care composition.

Preferably the personal care composition is a body wash or a shampoo.

A high shear in-line mixer is required in the preparation of a personal care composition from a composition from the process of the invention because in diluting the composition with water, the composition will pass through the highly viscous hexagonal phase before entering the low viscosity micellar phase.

### Brief Description of the Figure

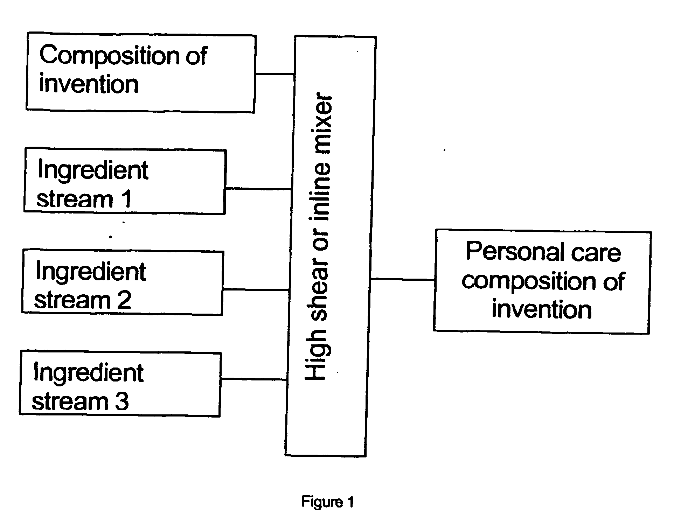

- Figure1: shows a schematic diagram of dilution with water of the composition to form a personal care composition.

The present invention will now be described further with reference to the following non-limiting examples. The percentages are all weight percentages and the temperatures are given in Celsius if not otherwise stated.

### Example 1 - 7: Compositions

The following compositions have been produced by using high shear mixing methods to incorporate the amphoteric surfactant into the mix. For some of the formulations such as those containing CMEA the mixtures are heated to 60°C and then subjected to high shear mixing. After these mixtures are homogenised they are cooled to room temperature under shear and then transferred to a container.

**Table 1: Compositions**

| Example | Sodium laureth sulfate (SLES) [weight (%)] | Cocamidopropyl betaine (CAPB) [weight (%)] | Cocomono ethanolamide (CMEA) [weight (%)] | Water [weight (%)] | pH |
|---|---|---|---|---|---|
| 1 | 66.1 | 6 | - | 27.9 | 6.5 |
| 2 | 62.5 | 11.4 | - | 26.1 | 6.2 |
| 3 | 59.3 | 16.2 | - | 24.5 | 6.3 |
| 4 | 56.4 | 20.5 | - | 23.1 | 6 |
| 5 | 41.8 | 41.8 | - | 16.4 | 5.8 |
| 6 | 38 | 45.6 | - | 16.4 | - |
| 7* | 63.3 | - | 9.2 | 27.2 | 7.6 |

| | | | | | |
|---|---|---|---|---|---|
| * not in accordance with the invention | | | | | |

### Examples 8 - 9: Personal Care Compositions

The following shower gels have been produced by making up the various other ingredients present in the shower gel into liquid ingredient streams. In some cases the liquid stream can also be pure water. These ingredient streams and the composition from the process according to the present invention are then combined together using a high shear in-line mixing process (sonolator or Silverson mixer) to arrive at a homogeneous shower gel. It is important to use a high shear inline mixer to handle the highly viscous hexagonal phase that is produced during the dilution process. The homogenisation of the hexagonal phase is difficult to handle with conventional mixing processes. This process in which the product is prepared by mixing various liquid containing streams to the composition also enables the shower gels to be customised or personalised by tuning the level and number of the individual ingredient streams that are added to the product. A schematic diagram for the dilution process is shown in figure 1.

### Example 8: Shower gel

| Ingredient | Weight (%) |
|---|---|
| Concentrate according to the present invention (composition of example 2) | 15.50 |
| Carbopol Aqua SF-1 (30% active) | 4.00 |
| EDTA NA | 0.10 |
| NaOH | 0.50 |
| Formalin @37% | 0.14 |
| Vacuum salt | 1.60 |
| Citric Acid | 0.14 |
| Perfume | 0.70 |
| Water | 74.64 |

### Example 9: Shower gel

| Ingredient | Weight (%) |
|---|---|
| Concentrate according to the present invention (composition of example 2) | 16.24 |
| Carbopol Aqua SF-1 | 5.00 |
| DMDM Hydantoin | 0.20 |
| NaOH (100%) | 0.11 |
| Citric Acid Monohydrate | 0.18 |
| Fragrance | 1.00 |
| Acusol OP302 | 1.50 |
| NaCl (100%) | 0.78 |
| Jaguar C13S | 0.15 |
| Water | 69.93 |

## Claims

1. A process for the manufacture of a composition, the composition comprising:
(i) 50 - 95 % by weight, based on the total weight of the composition, of
(ia) an alkyl ether sulphate and/or salt thereof, and
(ib) at least one further surfactant which is an anionic and/or amphoteric surfactant, and
(ic) optionally one or more non-ionic surfactants; and
(ii) 5 - 50 % by weight, based on the total weight of the composition, of water,
wherein the alkyl ether sulphate and/or salt thereof of (ia) and the anionic surfactant of (ib) are not identical surfactants, the process comprising the steps of:
(a) providing the alkyl ether sulphate or salt thereof in the form of a mixture with no more than 35% by weight, based on the total weight of the mixture, of water; then
(b) adding to and mixing with the mixture the at least one further surfactant chosen from the group consisting of anionic and/or amphoteric surfactants in substantially anhydrous form and the optional one or more non-ionic surfactants in substantially anhydrous form thereby to form the composition,
wherein the alkyl ether sulphate or salt thereof remains in a liquid crystalline lamellar phase or lamellar gel phase throughout the process.

2. A process for manufacturing a composition according to claim 1 comprising 50 - 90 %, preferably 60 - 85 % by weight of (i).

3. A process for manufacturing a composition according to any one of the preceding claims wherein the ratio of (ia) : [(ib) + (ic)] is 1:2 to 40:1, preferably 1:1 to 30:1.

4. A process for manufacturing a composition according to any one of the preceding claims comprising no more than 10 % by weight of (ic).

5. A process for manufacturing a composition according to any one of the preceding claims having a pH ≥ 5.

6. A process for manufacturing a composition according to any one of the preceding claims excluding added preservative.

7. A process for manufacturing a composition according to any one of the preceding claims excluding a pearlescent agent and/or an opacifying agent.

8. A process for manufacturing a composition according to any one of the preceding claims wherein (ia) is an alkyl ether sulphate salt of formula (I)
RO-(CH₂CH₂O)ₓSO₃M (I),
wherein R is an alkyl or alkenyl group of 8 to 24 carbon atoms,
x is an integer having a value of 1 to 10, and
M is a cation such as ammonium, alkanolamines such as triethanolamine, monovalent metals such as sodium and potassium, and polyvalent metal cations such as magnesium and calcium.

9. A process for manufacturing a composition according to claim 8 wherein (ia) is an alkyl ether sulphate salt selected from the group consisting of sodium and ammonium salts of coconut alkyl triethylene glycol ether sulphate, tallow alkyl diethylene glycol ether sulphate, tallow alkyl triethylene glycol ether sulphate and tallow alkyl hexaethylene sulphate.

10. A process for manufacturing a composition according to claim 9 wherein (ia) is sodium laureth sulphate.

11. A process for manufacturing a composition according to any one of the preceding claims wherein (ib) is an anionic surfactant selected from the group consisting of alkyl ether sulphate, alkyl sulphates, ethoxylated alkyl sulphates, alkyl ethoxycarboxylates, methyl acyl taurates, fatty acyl glycinates, N-acyl glutamates, acyl isethionates, alkyl sulphosuccinates, alkyl ethoxysulphosuccinates, alpha sulphonated fatty acids, esters of alpha sulphonated fatty acids, alkyl phosphate esters, ethoxylated alkyl phosphate esters, acyl sarcosinates, acyl aspartates, alkoxy cocamide carboxylates, (ethoxylated) alkanolamide sulphosuccinates, ethoxylated alkyl citrate sulphosuccinates, acyl ethylene diamine triacetates, acylhydroxyethyl isethionates, acyl amide alkoxy sulphates, linear alkyl benzene sulphonates, paraffin sulphonates, alpha olefin sulphonates, fatty acid/protein condensates, salts thereof, soaps such as ammonium, magnesium, potassium, triethanolamine and sodium salts of lauric acid, myristic acid and palmitic acid, and mixtures thereof and/or
an amphoteric surfactant which is a derivative of an aliphatic quaternary ammonium, phosphonium, or sulphonium compound, in which the aliphatic radicals can be straight or branched chain, and wherein one aliphatic substituent contains 8 to 18 carbon atoms and the same or different aliphatic substituent contains an anionic group such as a carboxy, a sulphonate, a sulphate, a phosphate or a phosphonate group.

12. A process for manufacturing a composition according to claim 11 wherein (ib) is cocamidopropyl betaine.

13. A process for manufacturing a composition according to any one of the preceding claims wherein (ic) is a non-ionic surfactant selected from the group consisting of alkyl poly(ethylene oxide) alkyl polyglucosides such as octyl glucoside and decyl maltoside, fatty alcohols such as cetyl alcohol and oleyl alcohol, cocamide MEA, cocamide DEA and cocamide TEA.

14. A process for manufacturing a composition according to any one of the preceding claims comprising at least one further additive.

15. A process for manufacturing a composition according to any one of the preceding claims wherein the mixture of alkyl ether sulphate or salt thereof and water comprises no more than 30%, preferably no more than 25% and more preferably no more than 20% by weight, based on the total weight of the mixture, of water.

## Patentansprüche

1. Verfahren zur Herstellung einer Zusammensetzung, wobei die Zusammensetzung umfasst:
(i) 50-95 Gewichts-%, bezogen auf das Gesamtgewicht der Zusammensetzung,
(ia) eines Alkylethersulfats und/oder eines Salzes davon und
(ib) wenigstens eines weiteren Tensids, das ein anionisches und/oder amphoteres Tensid ist, und
(ic) gegebenenfalls eines oder mehrerer nicht-ionischer Tenside und
(ii) 5-50 Gewichts-%, bezogen auf das Gesamtgewicht der Zusammensetzung, Wasser,
wobei das Alkylethersulfat und/oder Salz davon von (ia) und das anionische Tensid von (ib) nicht identische Tenside sind, wobei das Verfahren die Schritte:
(a) Bereitstellen des Alkylethersulfats oder eines Salzes davon in Form eines Gemisches mit nicht mehr als 35 Gewichts-%, bezogen auf das Gesamtgewicht des Gemisches, Wasser, danach
(b) Zusetzen des wenigstens einen weiteren Tensids, ausgewählt aus der Gruppe, bestehend aus anionischen und/oder amphoteren Tensiden, in im Wesentlichen wasserfreier Form und des optionalen einen oder der optionalen mehreren nicht-ionischen Tensid(e) in im Wesentlichen wasserfreier Form zu dem Gemisch und Mischen mit dem Gemisch, um dadurch die Zusammensetzung zu bilden, umfasst,
wobei das Alkylethersulfat oder Salz davon in einer flüssigkristallinen lamellaren Phase oder lamellaren Gelphase durch das Verfahren hindurch bleibt.

2. Verfahren zur Herstellung einer Zusammensetzung gemäß Anspruch 1, wobei die Zusammensetzung 50-90 Gewichts-%, vorzugsweise 60-85 Gewichts-%, an (i) umfasst.

3. Verfahren zur Herstellung einer Zusammensetzung gemäß Anspruch 1 oder Anspruch 2, wobei das Verhältnis von (ia): [(ib) + (ic)] 1:2 bis 40:1, vorzugsweise 1:1 bis 30:1, ist.

4. Verfahren zur Herstellung einer Zusammensetzung gemäß einem der vorangehenden Ansprüche, wobei die Zusammensetzung nicht mehr als 10 Gewichts-% an (ic) umfasst.

5. Verfahren zur Herstellung einer Zusammensetzung gemäß einem der vorangehenden Ansprüche, wobei die Zusammensetzung einen ph ≥ 5 hat.

6. Verfahren zur Herstellung einer Zusammensetzung gemäß einem der vorangehenden Ansprüche, wobei die Zusammensetzung ohne zugesetztes Konservierungsmittel ist.

7. Verfahren zur Herstellung einer Zusammensetzung gemäß einem der vorangehenden Ansprüche, wobei die Zusammensetzung keine Perlglanzmittel und/oder Trübungsmittel enthält.

8. Verfahren zur Herstellung einer Zusammensetzung gemäß einem der vorangehenden Ansprüche, wobei (ia) ein Alkylethersulfatsalz der Formel (I) ist
RO-(CH₂CH₂O)ₓSO₃M (I)
worin R eine Alkyl- oder Alkenyl-Gruppe mit 8 bis 24 Kohlenstoffatomen ist,
x eine ganze Zahl mit einem Wert von 1 bis 10 ist und
M ein Kation, zum Beispiel Ammonium, Alkanolamine, zum Beispiel Triethanolamin, einwertige Metalle, zum Beispiel Natrium und Kalium, und mehrwertige Metallkationen, zum Beispiel Magnesium und Calcium, ist.

9. Verfahren zur Herstellung einer Zusammensetzung gemäß Anspruch 8, wobei (ia) ein Alkylethersulfatsalz ist, das ausgewählt ist aus der Gruppe, bestehend aus Natrium- und Ammoniumsalzen von Kokosnussalkyltriethylenglykolethersulfat, Talgalkyldiethylenglykolethersulfat, Talgalkyltriethylenglykolethersulfat und Talgalkylhexaethylensulfat.

10. Verfahren zur Herstellung einer Zusammensetzung gemäß Anspruch 9, wobei (ia) Natriumlaurethsulfat ist.

11. Verfahren zur Herstellung einer Zusammensetzung gemäß einem der vorangehenden Ansprüche, wobei (ib) ein anionisches Tensid, ausgewählt aus der Gruppe, bestehend aus Alkylethersulfat, Alkylsulfaten, ethoxylierten Alkylsulfaten, Alkylethoxycarboxylaten, Methylacyltauraten, Fettacylglycinaten, N-Acylglutamaten, Acylisethionaten, Alkylsulfosuccinaten, Alkylethoxysulfosuccinaten, alpha-sulfonierten Fettsäuren, Estern von alpha-sulfonierten Fettsäuren, Alkylphosphatestern, ethoxylierten Alkylphosphatestern, Acylsarcosinaten, Acylaspartaten, Alkoxycocamidcarboxylaten, (ethoxylierten) Alkanolamidsulfosuccinaten, ethoxylierten Alkylcitratsulfosuccinaten, Acylethylendiamintriacetaten, Acylhydroxyethylisethionaten, Acylamidalkoxysulfaten, linearen Alkylbenzolsulfonaten, Paraffinsulfonaten, alpha-Olefinsulfonaten, Fettsäure/Protein-Kondensaten, Salzen davon, Seifen, zum Beispiel Ammonium-, Magnesium-, Kalium-, Triethanolamin- und Natriumsalze von Laurinsäure, Myristinsäure und Palmitinsäure, und Gemischen davon, und/oder ein amphoteres Tensid ist, welches ein Derivat einer aliphatischen quaternären Ammonium-, Phosphonium- oder Sulfonium-Verbindung ist, in der die aliphatischen Reste geradkettig oder verzweigtkettig sein können oder in der ein aliphatischer Substituent 8 bis 18 Kohlenstoffatome enthält und der gleiche oder verschiedene aliphatische Substituent eine anionische Gruppe, zum Beispiel eine Carboxy-, eine Sulfonat-, eine Sulfat-, eine Phosphat- oder eine PhosphonatGruppe, enthält.

12. Verfahren zur Herstellung einer Zusammensetzung gemäß Anspruch 11, wobei (ib) Cocamidopropylbetain ist.

13. Verfahren zur Herstellung einer Zusammensetzung gemäß einem der vorangehenden Ansprüche, wobei (ic) ein nicht-ionisches Tensid ist, ausgewählt aus der Gruppe, bestehend aus Alkylpoly(ethylenoxid), Alkylpolyglucosiden, zum Beispiel
Octylglucosid und Decylmaltosid, Fettalkoholen, zum Beispiel Cetylalkohol und Oleylalkohol, Cocamid-MEA, Cocamid-DEA und Cocamid-TEA.

14. Verfahren zur Herstellung einer Zusammensetzung gemäß einem der vorangehenden Ansprüche, wobei die Zusammensetzung wenigstens ein weiteres Additiv umfasst.

15. Verfahren zur Herstellung einer Zusammensetzung gemäß einem der vorangehenden Ansprüche, wobei das Gemisch aus Alkylethersulfat oder Salz davon und Wasser nicht mehr als 30 Gewichts-%, vorzugsweise nicht mehr als 25 Gewichts-% und bevorzugter nicht mehr als 20 Gewichts-%, bezogen auf das Gesamtgewicht des Gemisches, Wasser umfasst.

## Revendications

1. Procédé de fabrication d'une composition, la composition comprenant :
(i) 50 à 95 % en poids, par rapport au poids total de la composition, de
(ia) un alkyl éther sulfate et/ou un sel de celui-ci, et
(ib) au moins un agent tensioactif supplémentaire qui est un agent tensioactif anionique et/ou amphotère, et
(ic) facultativement un ou plusieurs agents tensioactifs non ioniques ; et
(ii) 5 à 50 % en poids, par rapport au poids total de la composition, d'eau,
où l'alkyl éther sulfate et/ou le sel de celui-ci de (ia) et l'agent tensioactif anionique (ib) ne sont pas des agents tensioactifs identiques, le procédé comprenant les étapes consistant à :
(a) fournir l'alkyl éther sulfate ou son sel sous la forme d'un mélange avec pas plus de 35 % en poids, par rapport au poids total du mélange, d'eau ; puis
(b) ajouter au mélange et mélanger avec celui-ci l'au moins un agent tensioactif supplémentaire choisi dans le groupe consistant en les agents tensioactifs anioniques et/ou amphotères sous forme sensiblement anhydre et les un ou plusieurs agents tensioactifs anioniques facultatifs sous forme sensiblement anhydre pour former ainsi la composition,
où l'alkyl éther sulfate ou son sel reste dans une phase lamellaire à cristaux liquides ou une phase de gel lamellaire tout au long du procédé.

2. Procédé de fabrication d'une composition selon la revendication 1, comprenant 50 à 90 %, de préférence, 60 à 85 % en poids de (i).

3. Procédé de fabrication d'une composition selon l'une quelconque des revendications précédentes, dans lequel le rapport de (ia) : [(ib) + (ic)] est de 1:2 à 40:1, de préférence, de 1:1 à 30:1.

4. Procédé de fabrication d'une composition selon l'une quelconque des revendications précédentes, comprenant pas plus de 10 % en poids de (ic).

5. Procédé de fabrication d'une composition selon l'une quelconque des revendications précédentes, dans lequel la composition a un pH ≥ 5.

6. Procédé de fabrication d'une composition selon l'une quelconque des revendications précédentes, excluant un conservateur ajouté.

7. Procédé de fabrication d'une composition selon l'une quelconque des revendications précédentes, excluant un agent perlescent et/ou un agent opacifiant.

8. Procédé de fabrication d'une composition selon l'une quelconque des revendications précédentes, dans lequel (ia) est un sel d'alkyl éther sulfate de formule (I)
RO-(CH₂CH₂O)ₓSO₃M (I),
dans laquelle R est un groupe alkyle ou alcényle de 8 à 24 atomes de carbone,
x est un nombre entier ayant une valeur de 1 à 10, et
M est un cation tel qu'un ammonium, des alcanolamines telles que la triéthanolamine, des métaux monovalents tels que le sodium et le potassium, et des cations de métaux polyvalents tels que le magnésium et le calcium.

9. Procédé de fabrication d'une composition selon la revendication 8, dans lequel (ia) est un sel d'alkyl éther sulfate choisi dans le groupe consistant en les sels de sodium et d'ammonium d'alkyle de noix de coco triéthylène glycol éther sulfate, alkyle de suif diéthylène glycol éther sulfate, alkyle de suif triéthylène glycol éther sulfate et alkyle de suif hexaéthylène sulfate.

10. Procédé de fabrication d'une composition selon la revendication 9, dans lequel (ia) est le laureth sulfate de sodium.

11. Procédé de fabrication d'une composition selon l'une quelconque des revendications précédentes, dans lequel (ib) est un agent tensioactif anionique choisi dans le groupe consistant en un alkyl éther sulfate, les alkyl sulfates, les alkyl sulfates éthoxylés, les éthoxycarboxylates d'alkyle, les acyl taurates de méthyle, les glycinates d'acyle gras, les N-acyl glutamates, les iséthionates d'acyle, les sulfosuccinates d'alkyle, les éthoxysulfosuccinates d'alkyle, les acides gras alpha sulfonatés, les esters d'acides gras alpha sulfonatés, les esters d'alkyl phosphate, les esters d'alkyl phosphate éthoxylés, les sarcosinates d'acyle, les aspartates d'acyle, les alcoxy cocamide carboxylates, les alcanolamide sulfosuccinates (éthoxylés), les alkyl citrate sulfosuccinates éthoxylés, les acyl éthylène diamine triacétates, les iséthionates d'acylhydroxyéthyle, les acyl amide alcoxy sulfates, les alkyl benzène sulfonates linéaires, les sulfonates de paraffine, les sulfonates d'alpha oléfine, les condensats acide gras/protéine, leurs sels, les savons tels que les sels d'ammonium, de magnésium, de potassium, de triéthanolamine et de sodium d'acide laurique, d'acide myristique et d'acide palmitique, et leurs mélanges et/ou
un agent tensioactif amphotère qui est un dérivé d'un composé ammonium, phosphonium ou sulfonium, quaternaire aliphatique, dans lequel les radicaux aliphatiques peuvent être à chaîne droite ou ramifiée, et où un substituant aliphatique contient 8 à 18 atomes de carbone et un substituant aliphatique identique ou différent contient un groupe anionique tel qu'un groupe carboxy, sulfonate, sulfate, phosphate ou phosphonate.

12. Procédé de fabrication d'une composition selon la revendication 11, dans lequel (ib) est une cocamidopropyl bétaïne.

13. Procédé de fabrication d'une composition selon l'une quelconque des revendications précédentes, dans lequel (ic) est un agent tensioactif non ionique choisi dans le groupe consistant en un alkyl poly(oxyde d'éthylène), les alkyl poly(glucosides) tels qu'octyl glucoside et décyl maltoside, les alcools gras tels que l'alcool cétylique et l'alcool oléylique, le cocamide MEA, le cocamide DEA et le cocamide TEA.

14. Procédé de fabrication d'une composition selon l'une quelconque des revendications précédentes, dans lequel la composition comprend au moins un additif supplémentaire.

15. Procédé de fabrication d'une composition selon l'une quelconque des revendications précédentes, dans lequel le mélange d'alkyl éther sulfate ou de son sel et de l'eau comprend pas plus de 30 %, de préférence pas plus de 25 % et de manière davantage préférée pas plus de 20 % en poids, par rapport au poids total du mélange, d'eau.
